# EUROPÄISCHE PATENTANMELDUNG

(11) **EP 1 184 007 A2**
(43) Veröffentlichungstag der Anmeldung: **06.03.2002**
(21) Anmeldenummer: 01250302.5
(22) Anmeldetag: 23.08.2001
(51) Int. Cl.: A61F 2/06

(54) **Spannungsoptimierter Stent**

(30) Priorität: 30.08.2000 DE 10044043
(71) Anmelder: BIOTRONIK Mess- und Therapiegeräte GmbH & Co Ingenieurbüro Berlin, 12359 Berlin (DE)
(72) Erfinder: Lootz, Daniel, 18119 Warnemünde (DE); Kranz, Curt, 14163 Berlin (DE)
(74) Vertreter: Eisenführ, Speiser & Partner

(57) **Zusammenfassung**

Stent, insbesondere peripherer Stent, zum Expandieren von einem ersten Zustand, in dem er in ein Gefäß (8; 8"') einführbar ist, in einen zweiten Zustand, in dem er das Gefäß (8; 8''') aufgeweitet hält, mit einer Anzahl ringförmiger Stützabschnitte (2, 2.1, 2.2) aus Stegelementen (3; 3'; 3"; 3'''), die in Längsrichtung des Stents (1; 1'; 1"; 1''') über Verbindungsstege (4; 4') verbunden sind, wobei die Stegelemente zumindest abschnittsweise aus einem eine Formgedächtnislegierung umfassenden Stentwerkstoff bestehen, dadurch gekennzeichnet, dass die Breite der Stegelemente (3; 3'; 3"; 3''') derart über ihre Länge variiert, dass die Spannungen, die in den Stegelementen (3; 3'; 3"; 3''') auftreten, wenn der Stentwerkstoff, insbesondere infolge einer Temperaturerhöhung, von einem ersten Gefügezustand in einen zweiten Gefügezustand übergeht, unterhalb der jeweiligen plastischen Verformungsgrenze des Stentwerkstoffs liegen.

## Beschreibung

Die vorliegende Erfindung betrifft einen Stent, insbesondere einen peripheren Stent, zum Expandieren von einem ersten Zustand, in dem er in ein Gefäß einführbar ist, in einen zweiten Zustand, in dem er das Gefäß aufgeweitet hält, mit einer Anzahl ringförmiger Stützabschnitte aus Stegelementen, die in Längsrichtung des Stents über Verbindungsstege verbunden sind, wobei die Stegelemente zumindest abschnittsweise aus einem eine Formgedächtnislegierung umfassenden Stentwerkstoff bestehen.

Es gibt zwei Arten von Stents. Zum einen die häufig als selbstexpandierend bezeichneten Stents, die in ihrem ersten Zustand von einer Hülleinrichtung umgeben und durch diese elastisch komprimiert sind, welche dann zum Expandieren vom Stent entfernt wird. Zum anderen gibt es die häufig als ballonexpansibel bezeichneten Stents, die auf einem expansiblen Ballon sitzen, der zur Expansion des Stents expandiert wird und dabei den Stent so weit plastisch verformt, dass dieser das Gefäß aufgeweitet hält.

Die selbstexpandierenden Stents werden häufig für periphere Anwendungen eingesetzt, beispielsweise im Bereich der Karotis-Arterien oder der Beinvenen. Gegenüber ballonexpansiblen Stents haben sie den Vorteil, dass sie aufgrund ihrer elastischen Eigenschaften nach einer ungewollten Verformung durch äußere mechanische Einflüsse, wie sie bei den peripheren Anwendungen durchaus auftreten können, von selbst wieder in den vollständig expandierten Zustand zurückkehren, in dem sie das Gefäß aufgeweitet halten.

Selbstexpandierende Stents werden in der Regel in einem sogenannten Kompressionskatheter in das Gefäß eingeführt, in dem sie auf einen verkleinerten Radius unter elastischer Verformung zusammengedrückt in einem Hüllrohr sitzen. An der Implantationsstelle angelangt, wird das Hüllrohr bezüglich des Stents zurückgezogen und dieser expandiert von selbst aufgrund der in ihm wirkenden elastischen Rückstellkräfte.

Solche selbstexpandierenden Stents haben jedoch den Nachteil, dass sie in der Regel nur mit relativ hohen Aufwand zu positionieren sind. Ihre korrekte Lage im expandierten Zustand ist vor dem Expandieren, d. h. solange sie sich in ihrem ersten Zustand im Hüllrohr befinden, nur schwer zu verifizieren. Einmal vollständig expandiert, lässt sich zwar die korrekte Positionierung des Stents gut überprüfen. Der Stent selbst lässt sich aber dann, wenn überhaupt, nur noch mit Mühe reponieren. Eine Korrektur der Lage des Stents ist somit kaum noch möglich.

Um dem Problem der korrekte Positionierung abzuhelfen, wird in der Europäischen Patentschrift EP 0 657 147 B1 im Zusammenhang mit einem in ähnlicher Weise selbstexpandierenden Gefäßimplantat vorgeschlagen, das expandierte Implantat beim Feststellen einer falschen Positionierung durch entsprechende Einrichtungen wieder in das Hüllrohr zurückzuziehen, um das Implantat erneut zu positionieren.

In diesem Zusammenhang hat es sich jedoch gezeigt, dass das Implantat bei mehrfach erforderlichem Zurückziehen in das Hüllrohr bleibende plastische Verformungen erfährt, welche seine Expansionsfähigkeit behindern. Dies ist jedoch im Interesse eines wirkungsvollen Eingriffs in jedem Fall zu vermeiden.

Ein vergleichbares Problem stellt sich im übrigen schon bei der erstmaligen Expansion des Implantats nach seiner erstmaligen Positionierung. Es hat sich nämlich gezeigt, dass die Expansionsfähigkeit beim erstmaligen Expandieren geringer ist als diejenige, die eigentlich nominell bestehen sollte.

Der vorliegenden Erfindung liegt daher die Aufgabe zu Grunde, eine einfache und zuverlässige Positionierung eines gattungsgemäßen Stents unter Beibehaltung der Expansionsfähigkeit des Stents zu ermöglichen.

Diese Aufgabe wird ausgehend von einem Stent gemäß dem Oberbegriff des Anspruchs 1 durch die im kennzeichnenden Teil des Anspruchs 1 angegebenen Merkmale gelöst.

Der Erfindung liegt die technische Lehre zu Grunde, dass man ein besonders einfaches und zuverlässiges Positionieren eines gattungsgemäßen Stents sicherstellt, wenn die Breite der Stegelemente derart über ihre Länge variiert, dass die Spannungen, die in den Stegelementen auftreten, wenn der Stentwerkstoff von einem ersten Gefügezustand in einen zweiten Gefügezustand übergeht, unterhalb der jeweiligen plastischen Verformungsgrenze des Stentwerkstoffs liegen.

Es hat sich gezeigt, dass durch eine entsprechende Variation der Breite der Stegelemente über ihre Länge erreicht werden kann, dass die Stegelemente bei dem Übergang von dem ersten in den zweiten Gefügezustand keine bleibende plastische Verformung erfahren. Die Variation der Stegbreite hat dabei den Vorteil, dass die Stegelemente des Stents ansonsten ihren auf die jeweiligen Eigenschaften des Stents abgestimmten Verlauf beibehalten können.

Der Übergang von dem ersten in den zweiten Gefügezustand kann aus einer Temperaturerhöhung resultieren. Dies kann beispielsweise vor dem erstmaligen Expandieren eines Stents der Fall sein, der bei einer Temperatur unterhalb der Körpertemperatur in einem ersten Gefügezustand in ein Hüllrohr eingeführt wurde und dann, z. B. beim Einführen in das Gefäß, auf Körpertemperatur gebracht wird, wodurch er in einen zweiten Gefügezustand übergeht. Ein solcher Fall liegt beispielsweise bei bevorzugten Varianten des erfindungsgemäßen Stents vor, bei denen der Übergang von dem ersten Gefügezustand in den zweiten Gefügezustand im ersten Zustand des Stents erfolgt.

Vorzugsweise handelt es sich dabei dann bei dem ersten Gefügezustand um einen martensitischen Zustand und bei dem zweiten Gefügezustand um einen spannungsinduzierten martensitischen Zustand.

Der Übergang von dem ersten in den zweiten Gefügezustand kann aber zusätzlich oder alternativ auch aus einer Veränderung der in Stent vorherrschenden Spannungen resultieren. Dies kann beispielsweise der Fall sein, wenn der Stent einmalig oder mehrfach aus einem zumindest teilweise expandierten Zustand in ein Hüllrohr oder dergleichen zurückgezogen wird, um seine Positionierung zu korrigieren.

Hierbei handelt es sich dann bei dem ersten Gefügezustand vorzugsweise um einen austenitischen Zustand und bei dem zweiten Gefügezustand um einen spannungsinduzierten martensitischen Zustand. Dies ist vorzugsweise dann der Fall, wenn sich der Stentwerkstoff bei Körpertemperatur im ersten Zustand des Stents in einem spannungsinduzierten martensitischen Zustand und im zweiten Zustand des Stents in einem austenitischen Zustand befindet.

Bei bevorzugten Varianten des erfindungsgemäßen Stents ist zusätzlich die Geometrie der Stegelemente derart gewählt, dass die Spannungen, die in den Stegelementen auftreten, wenn der Stentwerkstoff, beispielsweise infolge einer Temperaturerhöhung oder infolge einer Spannungsänderung, von dem ersten Gefügezustand in den zweiten Gefügezustand übergeht, unterhalb der jeweiligen plastischen Verformungsgrenze des Stentwerkstoffs liegen. Hierdurch ergibt sich eine zusätzliche Variationsmöglichkeit.

Besonders vorteilhaft lässt sich in die Erfindung wie erwähnt im Zusammenhang mit einem Stent einsetzen, der derart ausgebildet ist, dass er bezüglich einer zumindest abschnittsweise an ihm anliegenden Umhüllung in einer ersten Richtung ohne Verhaken an der Umhüllung versetzbar ist.

Besonders vorteilhaft ist dies, wenn sich der Stent bereits in einem zumindest abschnittsweise expandierten Zustand befindet und dann dennoch in der ersten Richtung bezüglich der Umhüllung versetzt werden kann.

Bei der Umhüllung kann es sich um eine gesonderte Hülleinrichtung, wie beispielsweise den Hüllkatheter eines selbstexpandierenden Stents oder einen entsprechenden Hüllkatheter für einen ballonexpansiblen Stent handeln. Sie kann aber auch von dem aufzuweitenden Gefäß selbst gebildet sein, welches dann gegebenenfalls unter Vorspannung an dem zumindest abschnittsweise expandierten Stent anliegt.

Bevorzugt wird die Erfindung im Zusammenhang mit den Varianten eingesetzt, bei denen eine Hülleinrichtung vorgesehen ist. So ist beispielsweise bei selbstexpandierenden Stents vorgesehen, dass dieser bei noch nicht vollständig vom Stent entfernter Hülleinrichtung durch Erzeugen einer Relativbewegung der Hülleinrichtung bezüglich des Stents in einer zur ersten Richtung entgegengesetzten zweiten Richtung ohne Verhaken an der Hülleinrichtung wieder in seinen ersten Zustand rückversetzbar ist.

Der Stent kann beispielsweise einfach in der ersten Richtung in die dann in ihrer Position gehaltene Hülleinrichtung zurückgezogen werden. Es kommt dabei zu keinem Verhaken oder vergleichbaren Effekten ähnlich dem sogenannten "Fishscaling" beim Einführen herkömmlicher ballonexpansibler Stents ohne Hüllkatheter.

Besonders vorteilhaft ist die erfindungsgemäße Gestaltung aber auch dann, wenn bei einem ballonexpansiblen Stent die erste Richtung als Einführrichtung des Stents an die Implantationsstelle verwendet wird, da es dann schon beim Einführen des Stents im nicht expandierten Zustand nicht zu dem besagten "Fishscaling", also dem Verhaken an der dann von dem Blutgefäß gebildeten Umhüllung kommen kann.

Vorzugsweise wird der Stent in seiner Position gehalten und die Hülleinrichtung bei selbstexpandierenden Stents erneut bzw. bei ballonexpansiblen Stents gegebenenfalls sogar erstmals über den Stent geschoben, um dabei eine möglichst geringe Belastung auf das Gefäß auszuüben.

Der Stent kann dank der erfindungsgemäßen Gestaltung zunächst im Bereich der Implantationsstelle expandiert werden und dann in situ durch herkömmliche Mittel hinsichtlich seiner korrekten Position bezüglich der Implantationsstelle überprüft werden. Das Expandieren kann dabei so erfolgen, dass der Stent schon über einen Großteil seiner gesamten Länge in seinen expandierten, zweiten Zustand übergegangen ist, bevor die Überprüfung der korrekten Positionierung erfolgt. Hierdurch wird erreicht, dass der Stent bei der Überprüfung schon weitestgehend seine tatsächliche expandierte Gestalt angenommen hat und daher bei Überprüfung eine genauere Beurteilung des späteren Sitzes des Stents vorgenommen werden kann.

Der Stent kann zum Verhindern des Verhakens der Stegelemente an der Umhüllung, beispielsweise an der Hülleinrichtung beim Rückversetzen in seinen ersten Zustand, mit einer Ummantelung aus einem Gewebe oder einer Folie versehen sein. Diese ist dann so ausgebildet, dass sie zum einen die Expansion des Stents ermöglicht. Zum anderen ist sie so gestaltet, dass über sie beim Rückversetzen des Stents auf in der ersten Richtung vorstehende Abschnitte der Stegelemente eine ausreichende radial nach innen gerichtete Kraftkomponente ausgeübt wird, welche sicherstellt, dass der Stent beim Rückversetzen in dem unmittelbar an das freie Ende der Hülleinrichtung angrenzenden Bereich jeweils schon so weit komprimiert, d. h. auf einen verringerten Durchmesser gebracht ist, dass er ohne Verhaken in die Hülleinrichtung gleiten bzw. diese über ihn gleiten kann. Das Gewebe bzw. die Folie müssen dabei lediglich so ausgestaltet sein, dass sie in Umfangsrichtung des Stents eine ausreichende Elastizität bzw. ein ausreichendes Übermaß gegenüber den Stent in seinem ersten Zustand aufweisen, welche die Expansion des Stents im Wesentlichen nicht behindern. In Längsrichtung des Stents hingegen ist eine ausreichend geringe Elastizität erforderlich, um die oben genannte Vorverformung in dem unmittelbar an das freie Ende der Hülleinrichtung angrenzenden Bereich des Stents bei dessen Rückversetzen in den ersten Zustand sicherzustellen.

Bevorzugt sind jedoch schon die Stegelemente und die Verbindungsstege selbst entsprechend ausgebildet und angeordnet, um das Verhaken zu verhindem. So greifen vorzugsweise die Verbindungsstege zwischen einem ersten ringförmigen Stützabschnitt und einem in der ersten Richtung benachbarten zweiten ringförmigen Stützabschnitt zum Verhindern eines Verhakens zwischen Stent und Hülleinrichtung beim Rückversetzen des Stents in seinen ersten Zustand im Bereich der in der ersten Richtung vorstehenden Abschnitte der Stegelemente des ersten ringförmigen Stützabschnitts an. Es kann bei diesen Varianten nicht zu einem Verhaken zwischen Stent und Hülleinrichtung kommen, da die Verbindungsstege dafür sorgen, dass in der ersten Richtung vorstehende Abschnitte der Stegelemente beim Rückversetzen des Stents in seinen ersten Zustand in dem unmittelbar das freie Ende der Einrichtung angrenzenden Bereich des Stents bereits so weit radial nach innen gezogen werden, dass diese ohne Verhaken in die Hülleinrichtung gleiten bzw. letztere über diese gleiten kann.

Eine besonders einfach herzustellende Ausgestaltung des erfindungsgemäßen Stents ergibt sich, wenn wenigstens ein erster ringförmiger Stützabschnitt und ein in der ersten Richtung benachbarter zweiter ringförmiger Stützabschnitt von jeweils einem in Umfangsrichtung des Stents mäanderförmig verlaufenden Stegelement gebildet sind und die Verbindungsstege zwischen dem ersten ringförmigen Stützabschnitt und dem zweiten ringförmigen Stützabschnitt im Bereich der an den zweiten Stützabschnitt angrenzenden Wendepunkte des Stegelements des ersten Stützabschnitts angreifen.

Vorzugsweise greift der jeweilige Verbindungssteg jeweils an dem am weitesten in der ersten Richtung vorstehenden Punkt des Stegelements des ersten ringförmigen Stützabschnitts an, da hierdurch sichergestellt ist, dass es in diesem Bereich keine Abschnitte des betreffenden Stegelements gibt, die in der ersten Richtung über diesen bei der oben genannten Vorverformung maßgebenden Kraftangriffspunkt hinausragen. Es kann somit zu keinem Verhaken beim Rückversetzen des Stents in seinen ersten Zustand kommen.

Bei bevorzugten Ausführungen des erfindungsgemäßen Stents greifen die Verbindungsstege bezüglich der Längsrichtung des Stents im Mittenbereich des zweiten ringförmigen Stützabschnitts an. Hierdurch ist sichergestellt, dass sich der Stent bei der Expansion möglichst wenig verkürzt, da eine starke Verkürzung bei der Expansion in der Regel unerwünscht ist. Es versteht sich jedoch, dass die Verbindungsstege auch an beliebigen anderen Stellen des zweiten ringförmigen Stützabschnitts, insbesondere auch an dessen Endbereichen bezüglich der Längsrichtung angreifen können.

In diesem Zusammenhang besonders vorteilhafte, weil einfach herzustellende Ausgestaltungen des erfindungsgemäßen Stents zeichnen sich dadurch aus, dass wenigstens der zweite ringförmige Stützabschnitt von einem in Umfangsrichtung des Stents mäanderförmig verlaufenden Stegelement gebildet ist und die Verbindungsstege bezüglich der Längsrichtung des Stents im Mittenbereich des Stegelements des zweiten Stützabschnitts zwischen den Wendepunkten des Stegelements des zweiten Stützabschnitts angreifen.

Bei weiteren vorteilhaften Varianten des erfindungsgemäßen Stents weisen die Verbindungsstege eine die Flexibilität des Stents bezüglich seiner Längsrichtung sicherstellende ausreichende Länge auf. Diese kann beispielsweise dadurch gewährleistet sein, dass der jeweilige Verbindungssteg nicht im Bereich des in Umfangsrichtung nächstliegenden, in der ersten Richtung vorstehenden Abschnitts des ersten Stegelements angreift, sondern im Bereich eines hierzu in Umfangsrichtung versetzten, entsprechend vorstehenden Abschnitts.

Weitere günstige Weiterbildungen des erfindungsgemäßen Stents zeichnen sich dadurch aus, dass die Verbindungsstege zur Vermeidung einer Verdrillung des Stents über seine Länge ausgebildet und angeordnet sind. Hierzu sind die Verbindungsstege in Längsrichtung des Stents vorzugsweise einzeln oder abschnittsweise wechselseitig bezüglich einer entlang der Längsrichtung des Stents verlaufenden Linien derart angeordnet, dass wenigstens ihren in der ersten Richtung liegenden Angriffspunkten an den Stegelementen bei der Expansion des Stents in der Tangentialebene des Stentmantels einzeln oder abschnittsweise eine Winkeländerung in entgegengesetzten Richtungen aufgeprägt wird. Diese entgegengesetzten Winkeländerungen bewirken, dass die ringförmigen Stützabschnitte bezüglich der Längsachse des Stents beim Expandieren einzeln oder abschnittsweise in Umfangsrichtung entgegengesetzt zueinander verdreht werden, wodurch sich über den gesamten Stent gesehen vorzugsweise eine vollständige Kompensation dieser Verdrehung ergibt, mithin also eine Verdrillung des Stents verhindert ist.

Bei bevorzugten, insbesondere selbstexpandierenden, Ausgestaltungen der erfindungsgemäßen Stents umfasst der Stentwerkstoff eine Formgedächtnislegierung. Hierbei kann es sich beispielsweise um ein superelastisches Material auf Kupferbasis handeln. Vorzugsweise wird jedoch auf Grund der guten physiologischen Verträglichkeit eine Nickel-Titan-Legierung verwendet. Diese Formgedächtnislegierungen haben den Vorteil, dass der Stent, ausgehend von einer ursprünglichen Form, bei einer ersten Temperatur plastisch verformt werden kann und bei einer Erhöhung der Temperatur dennoch wieder in seine ursprüngliche Form zurückkehrt.

Vorzugsweise befindet sich der Stentwerkstoff, wie oben erwähnt, bei Körpertemperatur im ersten Zustand des Stents in einem spannungsinduzierten martensitischen Zustand und im zweiten Zustand des Stents in einem austenitischen Zustand. Hierdurch ist es möglich, dass der Stent von einem Ausgangszustand, der im wesentlichen dem expandierten Endzustand entspricht, bei einer Temperatur unterhalb der Körpertemperatur so plastisch verformt, d. h. komprimiert werden kann, dass er bei dieser Temperatur ohne weiteres in die Hülleinrichtung eines entsprechenden Katheters eingeführt werden kann. Eine Erhöhung der Temperatur auf Körpertemperatur bewirkt, dass der Stent danach trachtet, wieder in seine ursprüngliche Form zurückzukehren. Hieran wird er zunächst durch die Hülleinrichtung gehindert, sodass er sich in einem spannungsinduzierten martensitischen Zustand befindet. Erst wenn die Hülleinrichtung entfernt wurde, expandiert der Stent, wobei er dann in seinen austenitischen Zustand übergeht.

Bevorzugte Varianten des erfindungsgemäßen Stents zeichnen sich, wie oben erwähnt, dadurch aus, dass die Breite der Stegelemente derart über ihre Länge variiert und gegebenenfalls zusätzlich die Geometrie der Stegelemente derart gewählt ist, dass die Spannungen, die in den Stegelementen auftreten, wenn der eine Formgedächtnislegierung umfassende Stentwerkstoff im ersten Zustand des Stents, beispielsweise infolge einer Temperaturerhöhung, vom martensitischen Zustand in einen spannungsinduzierten martensitischen Zustand übergeht, unterhalb der jeweiligen plastischen Verformungsgrenze des Stentwerkstoffs liegen.

Hierdurch ist sichergestellt, dass die selbstexpansiblen Eigenschaften des Stents nicht durch eine plastische Verformung des Stents während des Übergangs vom martensitischen in den spannungsinduzierten martensitischen Zustand beeinträchtigt werden. Dies ist insbesondere im Zusammenhang mit dem einmaligen oder mehrfachen Rückversetzen des Stents in seinen ersten Zustand von Vorteil. Andernfalls könnten dem Stent gerade beim mehrfachen Rückversetzen in seinen ersten Zustand fortschreitende plastische Verformungen aufgeprägt werden, die letztendlich zu einer nicht vollständigen Expansion des Stents führen könnten. Die Variation der Dicke der Stegelemente über ihre Länge stellt für Formgedächtnis-Stents, wie erwähnt, den hier zu Grunde liegenden Erfindungsgedanken dar. Es versteht sich weiterhin, dass vorzugsweise auch die Breite der Verbindungsstege in entsprechender Weise über deren Länge variiert.

Bei bevorzugten, weil einfach aufgebauten Varianten des erfindungsgemäßen Stents ist wenigstens ein ringförmiger Stützabschnitt von einem in Umfangsrichtung des Stents mäanderförmig verlaufenden Stegelement gebildet, dessen Breite zur Mitte zwischen zwei Wendepunkten hin abnimmt. Hierdurch kann in einfacher Weise die oben beschriebene Begrenzung der Spannungen im betreffenden Stegelement infolge der Temperaturerhöhung zum Übergang in den spannungsinduzierten martensitischen Zustand erzielt werden.

Weitere bevorzugte Ausführungen des erfindungsgemäßen Stents zeichnen sich dadurch aus, dass wenigstens ein ringförmiger Stützabschnitt von einem in Umfangsrichtung des Stents mäanderförmig verlaufenden Stegelement gebildet ist, dessen Krümmungsrichtung sich im Mittenbereich zwischen zwei im Verlauf des Stegelements benachbarten Wendepunkten ändert. Auch hierdurch wird eine günstige, weil gleichförmige Spannungsverteilung über das betreffende Stegelement erzielt.

Eine weitere in dem genannten Sinne vorteilhafte Beeinflussung der Spannungsverteilung über das betreffende Stegelement wird dadurch erzielt, dass wenigstens ein ringförmiger Stützabschnitt von einem in Umfangsrichtung des Stents mäanderförmig verlaufenden Stegelement gebildet ist, bei dem wenigstens die Mittellinie des Stegelements im Bereich der Wendepunkte die Form eines Ellipsenbogensegments aufweist.

Bei weiteren bevorzugten, weil besonders einfach aufgebauten Varianten des erfindungsgemäßen Stents ist wenigstens ein ringförmiger Stützabschnitt von einem in Umfangsrichtung des Stents mäanderförmig verlaufenden Stegelement gebildet, wobei je zwei in Umfangsrichtung des Stents benachbarte, zwischen den Wendepunkten verlaufende Stegelementabschnitte die Schenkel eines V bilden.

Die Erfindung betrifft weiterhin einen Katheter zum Implantieren eines erfindungsgemäßen Stents mit einem distalen Ende, in dessen Bereich eine Hülleinrichtung zur Aufnahme des Stents in seinem ersten Zustand vorgesehen ist, und einer Einrichtung zum Erzeugen der Relativbewegung zwischen Hülleinrichtung und Stent in der ersten Richtung. Erfindungsgemäß zeichnet sich diese Katheter dadurch aus, dass eine Einrichtung zum Erzeugen der Relativbewegung zwischen Hülleinrichtung und Stent in der zweiten Richtung und eine Halteeinrichtung zum Halten des Stents während dieser Relativbewegung in der zweiten Richtung vorgesehen sind. Hierdurch ist es in einfacher Weise möglich, den durch die Halteeinrichtung gehaltenen Stent in seinen ersten Zustand rückzuversetzen. Dies kann beispielsweise geschehen, indem der Stent bei festgehaltener Hülleinrichtung durch Verschieben der Halteeinrichtung bezüglich der Hülleinrichtung in letztere zurückgezogen wird. Alternativ kann natürlich auch der Stent über die Halteeinrichtung in seiner Position gehalten werden und die Hülleinrichtung mittels einer entsprechenden Einrichtung über den Stent geschoben werden.

Vorzugsweise sind ein Hüllrohr, dessen distales Ende die Hülleinrichtung bildet, und ein in diesem Hüllrohr zum Erzeugen der Relativbewegung in der ersten und zweiten Richtung verschieblich angeordnetes Halteelement zum Halten des Stents während der Relativbewegung in der zweiten Richtung vorgesehen. Hierdurch ergibt sich eine besonders einfache Konfiguration des Katheters.

Diese Katheter lassen sich sowohl mit selbstexpandierenden als auch mit ballonexpansiblen Stents einsetzen.

Bevorzugt ist ein erfindungsgemäßer Katheter bereits mit einem erfindungsgemäßen Stent versehen, der in der Hülleinrichtung des Katheters angeordnet ist.

Die vorliegende Erfindung betrifft weiterhin einen Verfahren zum Positionieren eines erfindungsgemäßen Stents in einem Gefäß. Hierbei kann es sich sowohl um ein Positionieren des Stents in vivo als auch in vitro, beispielsweise zu Prüfzwecken, handeln. Bei dem erfindungsgemäßen Verfahren wird beispielsweise der in einer Hülleinrichtung befindliche selbstexpandierende Stent in einem ersten Schritt in seinem ersten Zustand an die Expansionsstelle herangeführt. Anschließend wird der Stent in einem zweiten Schritt durch zumindest teilweises Entfernen der Hülleinrichtung vom Stent zumindest teilweise expandiert. In einem Überprüfungsschritt wird die Lage des Stents bezüglich der Expansionsstelle erfasst. Erfindungsgemäß ist dabei vorgesehen, dass in der Stent in dem zweiten Schritt nur teilweise expandiert wird. In wenigstens einem Korrekturschritt wird der Stent dann wieder in seinen ersten Zustand überführt, in dem er sich in der Hülleinrichtung befindet, und anschließend seine Position bezüglich der Expansionsstelle verändert. Dieser Korrekturschritt kann auch mehrfach wiederholt werden, bevor der Stent dann endgültig vollständig expandiert wird.

Dasselbe Verfahrensprinzip lässt sich auch mit einem ballonexpansiblen Stent durchführen, der zunächst gegebenenfalls zumindest über einen Teil seiner Länge ohne Hülleinrichtung an die Implantationsstelle herangeführt wird und dann unter Verwendung einer Hülleinrichtung in der oben beschriebenen Weise reponiert wird. Dabei wird der Stent in dem Korrekturschritt in einen dritten Zustand überführt, in dem er in der Hülleinrichtung angeordnet ist. Dieser dritte Zustand kann dem ersten Zustand entsprechen. Der Stent kann sich dabei aber auch gegenüber seinem ersten Zustand in einem vorzugsweise teilexpandierten Zustand, jedoch auch einem noch weiter komprimierten Zustand befinden.

Andere vorteilhafte Weiterbildungen der Erfindung ergeben sich aus den Unteransprüchen bzw. werden nachstehend zusammen mit der Beschreibung der bevorzugten Ausführungen der Erfindung anhand der beigefügten Figuren näher dargestellt. Es zeigen:
- Figur 1: die Abwicklung des Mantels einer bevorzugten Ausführungsform des erfindungsgemäßen Stents;
- Figur 2: einen schematischen Teilschnitt durch ein Ausführungsbeispiel eines erfindungsgemäßen Stents auf einem erfindungsgemäßen Katheter im ersten Zustand des Stents;
- Figur 3: einen Teilschnitt durch die Ausführung aus Figur 2 bei teilweise entfernter Hülleinrichtung;
- Figur 4: die Abwicklung des Mantels einer weiteren bevorzugten Ausführungsform des erfindungsgemäßen Stents;
- Figur 5: die Abwicklung eines Ausschnitts eines Stegelements gemäß einer weiteren bevorzugten Ausführungsform des erfindungsgemäßen Stents;
- Figur 6: einen Teilschnitt durch ein weiteres Ausführungsbeispiel eines erfindungsgemäßen Stents auf einem erfindungsgemäßen Katheter bei teilweise entfernter Hülleinrichtung.

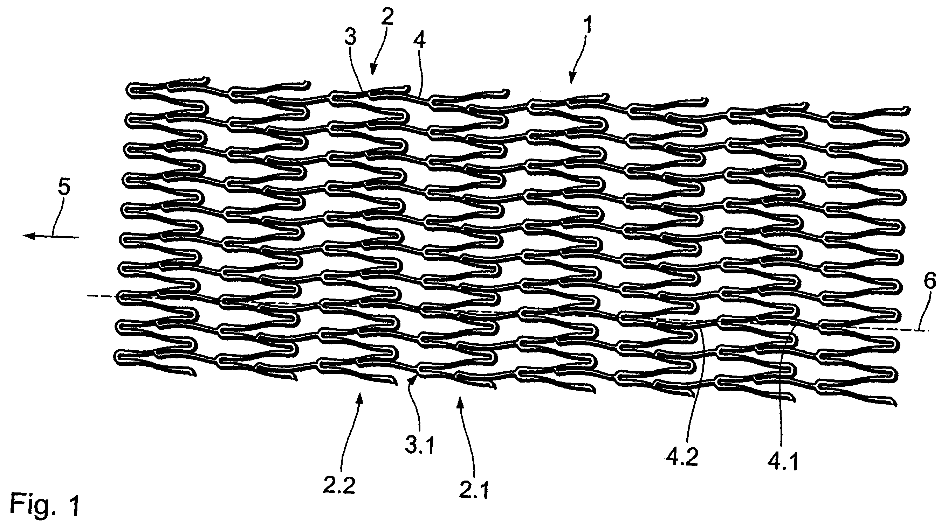

Figur 1 zeigt die Abwicklung des Mantels einer bevorzugten Ausführungsform des erfindungsgemäßen Stents 1 mit einer Anzahl ringförmiger Stützabschnitte 2 aus Stegelementen 3, die in Längsrichtung des Stents 1 über Verbindungsstege 4 miteinander verbunden sind. Der im nicht abgewickelten Zustand von einem dünnwandigen, rohrförmigen Bauteil gebildete Mantel des Stents 1 ist mit anderen Worten netzartig durchbrochen ausgebildet. Die Stegelemente 3 sowie die Verbindungsstege 4 sind dabei von der verbleibenden Wandung des rohrförmigen Bauteils gebildet.

Die ringförmigen Stützabschnitte 2 sind jeweils von einem in Umfangsrichtung des Stents 1 mäanderförmig verlaufenden Stegelement 3 gebildet. Die Verbindungsstege 4 zwischen einem ersten ringförmigen Stützabschnitt der 2.1 und einen zweiten ringförmigen Stützabschnitt 2.2 greifen jeweils im Bereich der in einer ersten Richtung 5 vorstehenden Abschnitte der Stegelemente 3 des ersten ringförmigen Stützabschnitts 2.1 an. Mit anderen Worten endet an jedem in der ersten Richtung 5 vorstehenden Wendepunkt 3.1 eines mäanderförmig verlaufenden Stegelements 3 ein Verbindungssteg 4 zu einem in der ersten Richtung 5 benachbarten Stegelement.

An den Stegelementen 3 des zweiten ringförmigen Stützabschnitts 2.2 greifen die Verbindungsstege 4 bezüglich der Längsrichtung des Stents jeweils im Mittenbereich zwischen den Wendepunkten des Stegelements 3 an. Hierdurch ist sichergestellt, dass sich der Stent 1 bei der Expansion von einem ersten Zustand, in dem er auf einen ersten Durchmesser komprimiert ist, in einen zweiten Zustand, in dem er auf einem größeren, zweiten Durchmesser expandiert ist, in seiner Längsrichtung nur minimal verkürzt. Dies rührt daher, dass sich bei dieser Konfiguration die Verkürzung des jeweiligen ringförmigen Stützabschnitts 2 bei der Expansion nur insoweit auswirkt, als lediglich die in Längsrichtung des Stents 1 erfolgende Lageänderung der Wendepunkte 3.1 bezüglich des genannten Mittenbereichs über die Verbindungsstege 4 zur Verkürzung des Stents 1 beiträgt. Ein weiterer Effekt dieser Gestaltung liegt darin, dass die Verbindungsstege 4 eine die Flexibilität des Stents 1 bezüglich seiner Längsrichtung sicherstellende Länge aufweisen. Der Stent 1 kann sich somit gut auch stark gekrümmten Gefäßen anpassen.

Die Verbindungsstege 4 sind weiterhin in Längsrichtung des Stents 1 bezüglich entlang der Längsrichtung des Stents 1 verlaufenden Linien 6 wechselseitig so angeordnet, dass dem in der ersten Richtung 5 liegenden Endpunkt bzw. Angriffspunkt eines ersten Verbindungsstegs 4.1 bei der Expansion des Stents 1 eine Winkeländerung aufgeprägt wird, die zur Winkeländerung entgegengesetzt ist, die hierbei dem in der ersten Richtung 5 liegenden Endpunkt bzw. Angriffspunkt eines in der ersten Richtung 5 benachbarten zweiten Verbindungsstegs 4.2 aufgeprägt wird. Hierdurch wird erreicht, dass sich die ringförmigen Stützabschnitte 2 des Stents 1 bei dessen Expansion zwar zueinander in seiner Umfangsrichtung verdrehen, sich diese Verdrehungen aber über die Länge des Stents 1 kompensieren. Hierdurch ist sichergestellt, dass der Stent 1 bei der Expansion über seine Länge keine nennenswerte Verdrillung erfährt.

Die Verdrillung ist dem gezeigten Beispiel durch die gewählte symmetrische Gestaltung und Anordnung der Verbindungsstege 4 vollständig ausgeschlossen. Es versteht sich jedoch, dass bei anderen Ausgestaltungen des erfindungsgemäßen Stents auch andere, asymmetrische Gestaltungen und Anordnungen der Verbindungsstege vorgesehen sein können, sofern sich mit diesen eine entsprechende Kompensation der Verdrehungen der ringförmigen Stützabschnitte ergibt.

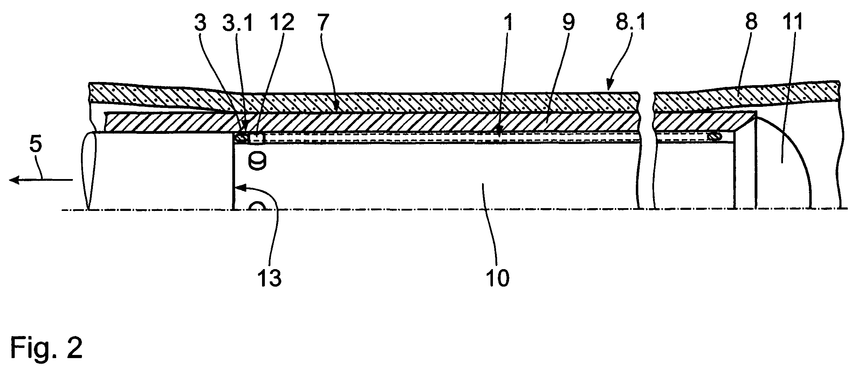

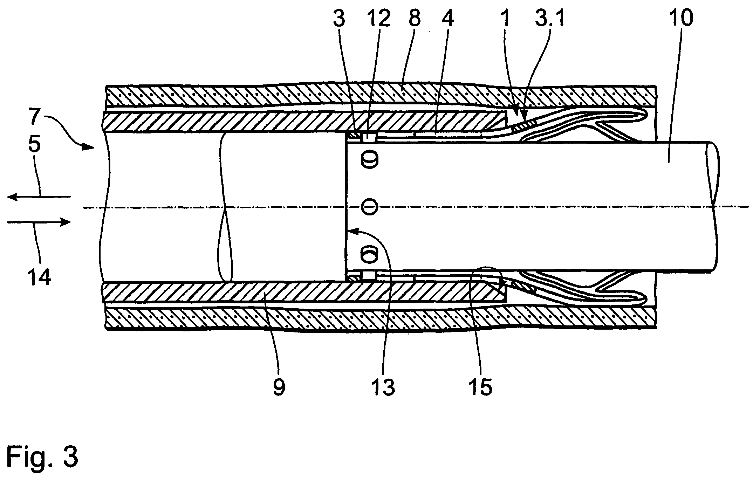

Bezug nehmend auf die Figuren 2 und 3 wird im Folgenden die Funktionsweise des erfindungsgemäßen Stents sowie des erfindungsgemäßen Katheters näher erläutert.

Figur 2 zeigt schematisch den Stent 1 aus Figur 1 in einem Teilschnitt auf einem Katheter 7, der in ein Blutgefäß 8 eingeführt ist. Der Stent 1 befindet sich dabei im Bereich einer Engstelle 8.1 dieses Blutgefäßes 8, die durch ihn aufgeweitet werden soll. Der Stent 1 ist in Figur 2 in seinem ersten Zustand dargestellt, in dem er sich vollständig in einer Hülleinrichtung des Katheters 7 befindet, die von einem am distalen Ende des Katheters 7 angeordneten Hüllrohr 9 gebildet ist. Der Stent 1 befindet sich dabei in einem auf einen verringerten Durchmesser komprimierten Zustand, sodass er auf Grund in ihm wirkender elastischer Rückstellkräfte gegen die Innenwandung des Hüllrohres 9 radial nach außen drückt.

Der Stent 1 ist auf einem Halter 10 angeordnet. Das Hüllrohr 9 und der Halter 10 sind verschieblich zueinander angeordnet. Am distalen Ende des Halters 10 befindet sich eine Abschlusskappe 11, die im gezeigten Zustand das Hüllrohr 9 verschließt, um das Einführen des Stents 1 in das Blutgefäß 8 zu erleichtern. Es versteht sich jedoch, dass diese Abschlusskappe bei anderen Varianten des erfindungsgemäßen Katheters nicht notwendigerweise vorgesehen sein muss.

Der Halter 10 weist Vorsprünge 12 auf, welche die am proximalen Ende des Stents 1 die Stegelemente 3 im Bereich der in der ersten Richtung 5 weisenden Wendepunkte 3.1 hintergreifen. Weiterhin weist der Halter 10 einen Absatz 13 auf, der als Anschlag für den Stent 1 im Bereich der genannten Wendepunkte 3.1 dient.

Um den Stent 1 in seinen zweiten - in Figur 2 nicht dargestellten - Zustand zu bringen, in dem er das Blutgefäß 8 aufgeweitet hält, kann das Hüllrohr 9 bezüglich des Halters 10 und damit auch bezüglich des Stents 1 in der ersten Richtung 5 zurückgezogen werden. Durch den Anschlag 13 wird dabei verhindert, dass der gegen die Innenwandung des Hüllrohrs 9 vorgespannte Stent 1 mit dem Hüllrohr 9 in der ersten Richtung 5 bewegt wird. Die Bereiche des Stents 1, die durch das Entfernen des Hüllrohrs 9 nicht mehr auf den verringerten Durchmesser komprimiert gehalten werden, expandieren umgehend. Unmittelbar nachdem das Hüllrohr 9 vollständig in der ersten Richtung 5 von den Stent 1 abgezogen ist, ist der Stent 1 über seine ganze Länge expandiert und befindet sich somit in seinem zweiten Zustand.

Figur 3 zeigt einen weiteren Teilschnitt durch die Ausführung aus Figur 2 in einem Zustand des Stents 1, in dem das Hüllrohr 9 teilweise von dem Stent 1 entfernt, d. h. in der ersten Richtung 5 bezüglich des Stents 1 zurückgezogen ist. Die Abschnitte des Stents 1, die außerhalb des Hüllrohrs 9 liegen sind bereits weitgehend auf ihren Enddurchmesser expandiert. Lediglich in dem Bereich, der unmittelbar an das distale Ende des Hüllrohrs 9 angrenzt, findet in Längsrichtung des Stents 1 ein langsamer Übergang von dem komprimierten Durchmesser auf den expandierten Durchmesser statt.

Wie Figur 3 zu entnehmen ist, befindet sich nur noch ein kleiner Teil des Stents 1 im Hüllrohr 9. Folglich ist bereits ein Großteil Stents 1 vollständig expandiert. In diesem Zustand erhält man bei der Überprüfung der Positionierung des Stents 1 bezüglich der Engstelle 8.1 im Blutgefäß 8 ein weitestgehend unverfälschtes Bild von der späteren Position des vollständig expandierten Stents 1, da sich bei der weiteren Expansion des kleinen Restes des Stents 1, der noch im Hüllrohr 9 verblieben ist, keine wesentliche Änderung der Lage des Stents mehr ergibt.

Gemäß dem erfindungsgemäßen Verfahren kann nunmehr ein Korrekturschritt vorgenommen werden, bei dem der teilexpandierte Stent wieder in seinen ersten Zustand rückversetzt wird und dann seine Position korrigiert wird. Es versteht sich dabei, dass dieses Verfahren nicht notwendigerweise in vivo, d. h. am Patienten ausgeführt werden muss. Es kann auch in vitro, d. h. an beliebigen anderen Gefäßen oder dergleichen ausgeführt werden.

Das Rückversetzen des Stents 1 erfolgt, indem das Hüllrohr 9 relativ zum Halter 10 mittels einer - in den Figuren 2 und 3 nicht gezeigten - Einrichtung am proximalen Ende des Katheters 7 in einer zur ersten Richtung entgegengesetzten zweiten Richtung 14 wieder über den Stent 1 geschoben wird. Die Vorsprünge 12, welche die Stegelemente 3 am proximalen Ende des Stents 1 hintergreifen, halten den Stent 1 in seiner Position bezüglich des Halters 10 und stellen so sicher, dass der Stent durch das vorlaufende, distale Ende des Hüllrohrs 9 wieder in seinen komprimierten Zustand gebracht wird, wie er in Figur 2 dargestellt ist.

Die Gestaltung und Anordnung der Stegelemente 3 und der Verbindungsstege 4 stellt dabei sicher, dass es zu keinem Verhaken der in der ersten Richtung 5 vorstehenden Abschnitte, also der in der ersten Richtung 5 vorstehenden Wendepunkte 3.1 der Stegelemente 3 an dem verlaufenden, distalen Ende des Hüllrohrs 9 kommen kann. Die Verbindungsstege 4 sorgen hierbei dafür, dass die in der ersten Richtung vorstehenden Wendepunkte 3.1 der Stegelemente 3 beim Überschieben des Hüllrohrs 9 über den Stent 1 in dem unmittelbar an das vorlaufende Ende des Hüllrohrs 9 angrenzenden Bereich des Stents 1 bereits so weit radial nach innen gezogen werden, dass das Hüllrohr 9 ohne Verhaken über die in der ersten Richtung 5 vorstehenden Abschnitte der Stegelemente 3 gleiten kann. Das reibungslose Überschieben des Hüllrohrs 9 wird dabei noch durch eine Fase 15 am distalen Ende des Hüllrohrs 9 unterstützt.

Dem gezeigten Beispiel greifen die Verbindungsstege 4 unmittelbar im Bereich der in der ersten Richtung 5 weisenden Wendepunkte 3.1 der Stegelemente 3 an. Mit anderen Worten greifen die Verbindungsstege 4 unmittelbar an dem am weitesten in der ersten Richtung 5 vorstehenden Abschnitt des jeweiligen Stegelements 3 an. Es versteht sich jedoch, dass die Verbindungsstege bei anderen Ausführungen des erfindungsgemäßen Stents nicht notwendigerweise an diesem am weitesten in der ersten Richtung vorstehenden Abschnitt des jeweiligen Stegelement angreifen müssen. Sie können in deren Bereich auch an einem weniger weit in der ersten Richtung vorstehenden Abschnitt des jeweiligen Stegelements angreifen. Mit anderen Worten kann der Angriffspunkt des jeweiligen Verbindungsstegs in der ersten Richtung von angrenzenden Abschnitten des Stegelements noch überragt werden. Es muss lediglich sichergestellt sein, dass diese den Angriffspunkt in der ersten Richtung überragenden Abschnitte des Stegelements beim Rückversetzen des Stents in seinem ersten Zustand über die Verbindungsstege so weit radial nach ihnen gezogen werden, dass die Hülleinrichtung, beispielsweise also das Hüllrohr, ohne Verhaken über diese Abschnitte gleiten kann.

Es versteht sich weiterhin, dass das genannte Prinzip, in der ersten Richtung vorstehende Abschnitte der Stegelemente über die jeweiligen Verbindungsstege so weit radial nach innen zu ziehen, dass die Hülleinrichtung ohne Verhaken über diese Abschnitte gleiten kann, nicht auf die in Figur 1 gezeigten mäanderförmigen Stegelemente beschränkt ist, sondern sich auch auf beliebige, anders verlaufende Stegelemente angewandt werden kann.

Es versteht sich weiterhin, dass sich das eben beschriebene Verfahrensprinzip auch mit einem ballonexpansiblen Stent durchführen lässt, der auf einem entsprechenden Ballon sitzend in der Hülleinrichtung an die Implantationsstelle herangeführt wird, dann durch den Ballon teilweise expandiert wird, d. h. in den in Figur 3 dargestellten Zustand gebracht wird, und unter Verwendung der Hülleinrichtung in der oben beschriebenen Weise reponiert wird.

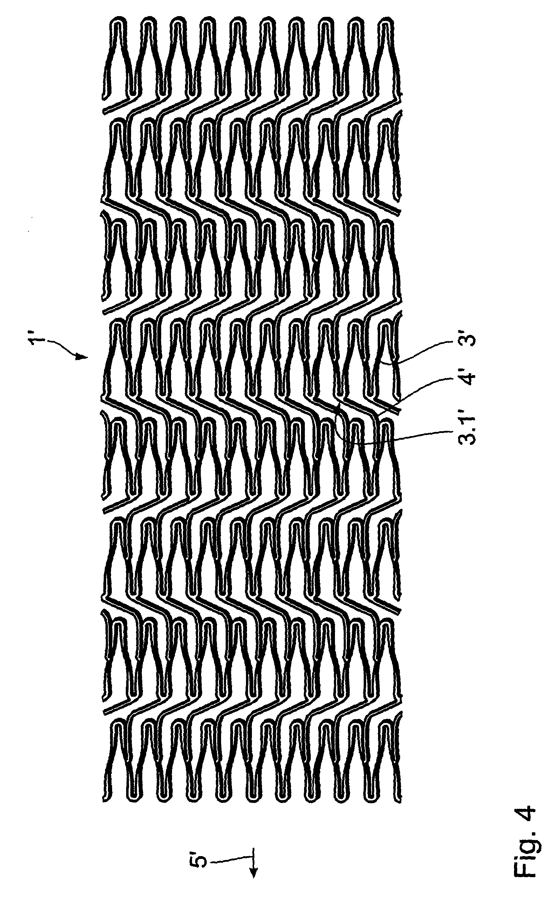

Figur 4 zeigt die Abwicklung des Mantels einer anderen Ausführungsform des erfindungsgemäßen Stents. Diese gleicht in ihrem grundsätzlichen Aufbau derjenigen aus Figur 1, sodass hier lediglich auf die Unterschiede eingegangen werden soll.

Der Unterschied besteht darin, dass die Verbindungsstege 4' nicht unmittelbar an dem in Längsrichtung des Stents 1' nächstliegenden Wendepunkt 3.1' des Stegelements 3' angreifen, sondern an einem hierzu in Umfangsrichtung des Stents 1' versetzten Wendepunkt 3.1'. Hierdurch erhöht sich die Länge der Verbindungsstege 4' gegenüber der Ausführung aus Figur 1, was wiederum zu einer Erhöhung der Flexibilität des Stents 1' bezüglich seiner Längsrichtung führt.

Die Stents aus den Figuren 1 bis 4 bestehen jeweils aus einer Formgedächtnislegierung auf Nickel-Titan-Basis, so genanntem Nitinol. Dieser Stentwerkstoff befindet sich bei Körpertemperatur im ersten Zustand des Stents 1, also in seinem im Hüllrohr 9 komprimierten Zustand, in einem spannungsinduzierten martensitischen Zustand. Im zweiten Zustand, also weitgehend entspannten Zustand des Stents 1 befindet er sich in einem austenitischen Zustand. Bei der Fertigung bzw. vor seiner Anwendung wird der Stent 1 von einem Ausgangszustand, der im wesentlichen dem expandierten Endzustand entspricht, bei einer Temperatur, die unterhalb der Körpertemperatur liegt und bei der er sich in einem martensitischen Zustand befindet, so plastisch verformt, d. h. komprimiert, dass er bei dieser Temperatur ohne weiteres in das Hüllrohr 9 des Katheters 7 eingeführt werden kann. Eine Erhöhung der Temperatur auf Körpertemperatur bewirkt dann, dass der Stent danach trachtet, wieder in seine ursprüngliche Form zurückzukehren. Hieran wird er zunächst durch das Hüllrohr 9 gehindert, sodass er sich in einem spannungsinduzierten martensitischen Zustand befindet. Erst wenn das Hüllrohr 9 entfernt wird, expandiert der Stent 1 und gelangt so in seinen austenitischen Zustand.

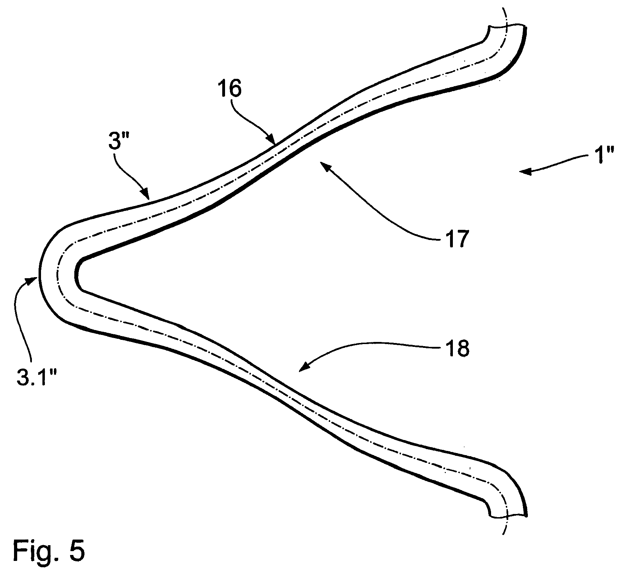

Der Dickenverlauf und die Geometrie der Stegelemente aus den Figuren 1 bis 4 entsprechen denjenigen, die im folgenden im Zusammenhang mit Figur 5 beschrieben werden.

Figur 5 zeigt die Abwicklung eines Ausschnitts eines Stegelements 3" gemäß einer bevorzugten Ausführung des erfindungsgemäßen Stents. Auch hier handelt es sich um eine Ausführung aus einer der oben beschriebenen Formgedächtnislegierungen. Der Stent aus Figur 5 kann im wesentlichen den Stents aus den Figuren 1 bis 4 entsprechen, sodass hier lediglich auf die Besonderheiten der Stegelemente eingegangen werden soll

Die Besonderheit des Stegelements 3" liegt darin, dass zum einen seine Geometrie so gewählt ist und zum anderen die Breite des Stegelements 3" über seine Länge derart variiert, dass die Spannungen, die in ihm auftreten, wenn der Stentwerkstoff im ersten Zustand des Stents 1" infolge einer Temperaturerhöhung vom martensitischen Zustand in einen spannungsinduzierten martensitischen Zustand übergeht, unterhalb der bei der jeweiligen Temperatur vorherrschenden plastischen Verformungsgrenze des Stentwerkstoffs bleiben.

Diese günstige Spannungsverteilung wird zum einen dadurch erzielt, dass die Breite des Stegelements 3" jeweils zur Mitte 16 zwischen zwei Wendepunkten 3.1" hin kontinuierlich abnimmt. Die Dickenabnahme beträgt beim gezeigten Beispiel etwa 50%. Sie bestimmt sich jedoch bei anderen Gestaltungen des Stegelements in Abhängigkeit von der sonstigen Geometrie des Stegelements nach der jeweils einzuhaltenden Spannungsobergrenze.

Eine weitere vorteilhafte Beeinflussung der Spannungsverteilung innerhalb des Stegelements 3" ergibt sich dadurch, dass sich die Krümmungsrichtung des Stegelements 3" im Mittenbereich 16 zwischen zwei Wendepunkten 3.1" ändert. Je zwei in Umfangsrichtung des Stents 1" benachbarte, zwischen den Wendepunkten 3.1" verlaufende Stegelementabschnitte 17 und 18 bilden somit die geschwungenen Schenkel eines V.

Eine zusätzliche in dem genannten Sinne vorteilhafte Beeinflussung der Spannungsverteilung über das Stegelement 3" ergibt sich dadurch, dass das Stegelement 3" im Bereich der Wendepunkte 3.1" an Stelle des üblichen Kreisbogensegments die Form eines Ellipsenbogensegments aufweist.

Es versteht sich, dass das grundlegende Prinzip, den Breitenverlauf und gegebenenfalls zusätzlich die Geometrie der Stegelemente so zu wählen, dass die Spannungen im Stegelement unterhalb der jeweiligen plastischen Verformungsgrenze beim Übergang vom martensitischen Zustand in einen spannungsinduzierten martensitischen Zustand bleiben, unabhängig von den zu den Figuren 1 bis 5 beschriebenen grundsätzlichen Geometrien der Stegelemente auch beliebige andere Verläufe der Stegelemente anwendbar ist.

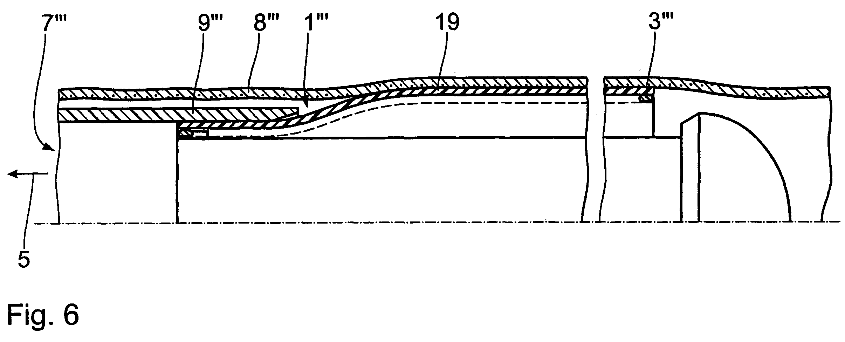

Figur 6 zeigt einen Teilschnitt durch eine weitere Ausführungsform eines erfindungsgemäßen Stents auf einem erfindungsgemäßen Katheter 7''' im teilexpandierten Zustand. Der Katheter 7''' entspricht dabei im Wesentlichen dem Katheter aus den Figuren 2 und 3, sodass hier lediglich auf die Unterschiede bezüglich des Stents 1''' eingegangen werden soll.

Der Unterschied besteht darin, dass der Stent 1''' zum Verhindern des Verhakens der Stegelemente 3''' am Hüllrohr 9''' beim Rückversetzen in seinen ersten Zustand mit einer Ummantelung 19 versehen ist. Diese Ummantelung 19 ist so ausgebildet, dass, sie zum einen die Expansion des Stents 1''' bis in dessen gewünschten Endzustand ermöglicht. Zum anderen ist sie so ausgebildet, dass über sie beim Rückversetzen des Stents 1''' auf in der ersten Richtung 5''' vorstehende Abschnitte der Stegelemente 3''' eine ausreichende radial nach innen gerichtete Kraftkomponente ausübt, welche sicherstellt, dass der Stent 1''' beim Rückversetzen in dem unmittelbar an das freie, distale Ende des Hüllrohrs 9''' angrenzenden Bereich, jeweils schon so weit komprimiert, d. h. auf einen verringerten Durchmesser gebracht ist, dass das Hüllrohr 9''' ohne Verhaken über den Stent 1''' gleiten kann.

Die Ummantelung 19 weist hierzu in Umfangsrichtung des Stents eine ausreichende Elastizität auf, welche die Expansion des Stents im Wesentlichen nicht behindert. In Längsrichtung des Stents hingegen besitzt sie hingegen eine geringe Elastizität, um die oben genannte Vorverformung in dem unmittelbar an das freie Ende der des Hüllrohrs angrenzenden Bereich des Stents 1''' bei dessen Rückversetzen in den ersten Zustand sicherzustellen. Im gezeigten Beispiel ist dies durch eine Folie 19 aus entsprechend elastischem Kunststoff realisiert, in die in Längsrichtung des Stents verlaufende, entsprechend zugfeste Fasern eingebettet sind. Die Zugfasern verlaufen im Bereich der in der ersten Richtung 5''' vorstehenden Abschnitte der Stegelemente 3'''. In diesem Bereich sind die Stegelemente 3''' auch mit der Folie 19 verbunden, um eine gleichmäßige Krafteinleitung zu gewährleisten bzw. ein Abgleiten der Fasern von diesen Bereichen der Krafteinleitung zu verhindern.

Der zweite Stoff kann aus einer oder mehreren Komponenten bestehen, die mit keiner der Komponenten des ersten Stoffes stoffgleich ist bzw. sind.

Der zweite Stoff kann aber ebenso gut einer Komponente des ersten Stoffes entsprechen. Bei einem Aufbau des ersten Stoffes aus einer Matrix, in der die erste Substanz eingelagert ist, kann der zweite Stoff beispielsweise aus der die Matrix bildenden Komponente des ersten Stoffes bestehen bzw. dieser entsprechen.

## Patentansprüche

1. Stent, insbesondere peripherer Stent, zum Expandieren von einem ersten Zustand, in dem er in ein Gefäß (8; 8''') einführbar ist, in einen zweiten Zustand, in dem er das Gefäß (8; 8''') aufgeweitet hält, mit einer Anzahl ringförmiger Stützabschnitte (2, 2.1, 2.2) aus Stegelementen (3; 3'; 3"; 3'''), die in Längsrichtung des Stents (1; 1'; 1"; 1''') über Verbindungsstege (4; 4') verbunden sind, wobei die Stegelemente zumindest abschnittsweise aus einem eine Formgedächtnislegierung umfassenden Stentwerkstoff bestehen, **dadurch gekennzeichnet, dass** die Breite der Stegelemente (3; 3'; 3"; 3''') derart über ihre Länge variiert, dass die Spannungen, die in den Stegelementen (3; 3'; 3"; 3''') auftreten, wenn der Stentwerkstoff, insbesondere infolge einer Temperaturerhöhung, von einem ersten Gefügezustand in einen zweiten Gefügezustand übergeht, unterhalb der jeweiligen plastischen Verformungsgrenze des Stentwerkstoffs liegen.

2. Stent nach Anspruch 1, **dadurch gekennzeichnet, dass** der erste Gefügezustand ein martensitischer Zustand ist und der zweite Gefügezustand ein spannungsinduzierter martensitischer Zustand ist.

3. Stent nach Anspruch 1 oder 2, **dadurch gekennzeichnet, dass** der Übergang von dem ersten Gefügezustand in den zweiten Gefügezustand im ersten Zustand des Stents (1; 1'; 1"; 1'") erfolgt.

4. Stent nach einem der vorhergehenden Ansprüche, **dadurch gekennzeichnet, dass** der Stentwerkstoff eine Nickel-Titan-Legierung umfasst.

5. Stent nach einem der vorhergehenden Ansprüche, **dadurch gekennzeichnet, dass** sich der Stentwerkstoff bei Körpertemperatur im ersten Zustand des Stents (1; 1'; 1"; 1''') in einem spannungsinduzierten martensitischen Zustand und im zweiten Zustand des Stents (1; 1'; 1"; 1''') in einem austenitischen Zustand befindet.

6. Stent nach einem der vorhergehenden Ansprüche, **dadurch gekennzeichnet, dass** die Geometrie der Stegelemente (3; 3'; 3"; 3''') derart gewählt ist, dass die Spannungen, die in den Stegelementen (3; 3'; 3"; 3''') auftreten, wenn der Stentwerkstoff, insbesondere infolge einer Temperaturerhöhung, von dem ersten Gefügezustand in den zweiten Gefügezustand übergeht, unterhalb der jeweiligen plastischen Verformungsgrenze des Stentwerkstoffs liegen.

7. Stent nach einem der vorhergehenden Ansprüche, **dadurch gekennzeichnet, dass** wenigstens ein ringförmiger Stützabschnitt von einem in Umfangsrichtung des Stents (1; 1'; 1"; 1''') mäanderförmig verlaufenden Stegelement gebildet ist, dessen Breite zur Mitte (16) zwischen zwei Wendepunkten (3.1; 3.1'; 3.1") hin abnimmt.

8. Stent nach einem der vorhergehenden Ansprüche, **dadurch gekennzeichnet, dass** wenigstens ein ringförmiger Stützabschnitt (2, 2.1, 2.2) von einem in Umfangsrichtung des Stents (1; 1'; 1"; 1''') mäanderförmig verlaufenden Stegelement (3; 3'; 3"; 3''') gebildet ist, dessen Krümmungsrichtung sich im Mittenbereich (16) zwischen zwei Wendepunkten (3.1; 3.1'; 3.1") ändert.

9. Stent nach einem der vorhergehenden Ansprüche, **dadurch gekennzeichnet, dass** wenigstens ein ringförmiger Stützabschnitt (2, 2.1, 2.2) von einem in Umfangsrichtung des Stents (1; 1'; 1"; 1''') mäanderförmig verlaufenden Stegelement (3; 3'; 3"; 3''') gebildet ist, wenigstens dessen Mittellinie im Bereich der Wendepunkte (3.1; 3.1'; 3.1") die Form eines Ellipsenbogensegments aufweist.

10. Stent nach einem der vorhergehenden Ansprüche, **dadurch gekennzeichnet, dass** wenigstens ein ringförmiger Stützabschnitt (2, 2.1, 2.2) von einem in Umfangsrichtung des Stents (1; 1'; 1"; 1''') mäanderförmig verlaufenden Stegelement (3; 3'; 3''; 3''') gebildet ist, wobei je zwei in Umfangsrichtung des Stents (1; 1'; 1"; 1''') benachbarte, zwischen den Wendepunkten (3.1; 3.1'; 3.1") verlaufende Stegelementabschnitte (17, 18) die Schenkel eines V bilden.

11. Stent nach einem der vorhergehenden Ansprüche, **dadurch gekennzeichnet, dass** die Stegelemente (3; 3'; 3") und die Verbindungsstege (4; 4') derart ausgebildet und angeordnet sind, dass der Stent (1; 1'; 1") bezüglich einer zumindest abschnittsweise an ihm anliegenden Umhüllung (8, 9; 8'''; 9'''), insbesondere in einem zumindest abschnittsweise expandierten Zustand, in der ersten Richtung (5; 5'; 5"; 5''') ohne Verhaken an der Umhüllung (8, 9; 8'''; 9''') versetzbar ist.

12. Stent nach Anspruch 11, **dadurch gekennzeichnet, dass** die Verbindungsstege (4; 4') zwischen einem ersten ringförmigen Stützabschnitt (2.1) und einem in der Versatzrichtung (5; 5') benachbarten zweiten ringförmigen Stützabschnitt (2.2) zum Verhindern eines Verhakens zwischen Stent (1; 1') und Umhüllung (8; 9) beim Versetzen des Stents (1; 1') im Bereich der in der ersten Richtung (5; 5') vorstehenden Abschnitte der Stegelemente (3; 3') des ersten ringförmigen Stützabschnitts (2.1) angreifen.

13. Stent nach Anspruch 11 oder 12, **dadurch gekennzeichnet, dass** wenigstens ein erster ringförmiger Stützabschnitt (2.1) und ein in der ersten Richtung (5; 5') benachbarter zweiter ringförmiger Stützabschnitt (2.2) von jeweils einem in Umfangsrichtung des Stents (1; 1') mäanderförmig verlaufenden Stegelement (3; 3') gebildet sind und die Verbindungsstege (4; 4') zwischen dem ersten ringförmigen Stützabschnitt (2.1) und dem zweiten ringförmigen Stützabschnitt (2.2) im Bereich der an den zweiten Stützabschnitt (2.2) angrenzenden Wendepunkte des Stegelements (3; 3') des ersten Stützabschnitts (2.1) angreifen.

14. Stent nach einem der Ansprüche 12 oder 13, **dadurch gekennzeichnet, dass** die Verbindungsstege (4; 4') bezüglich der Längsrichtung des Stents (1; 1') im Mittenbereich des zweiten ringförmigen Stützabschnitts (2.2), insbesondere im Mittenbereich des Stegelements (3; 3') des zweiten Stützabschnitts (2.2) zwischen den Wendepunkten (3.1; 3.1') des Stegelements (3; 3'), angreifen.

15. Stent nach einem der vorhergehenden Ansprüche, **dadurch gekennzeichnet, dass** die Verbindungsstege (4; 4') eine die Flexibilität des Stents (1; 1') bezüglich seiner Längsrichtung sicherstellende ausreichende Länge aufweisen und/oder zur Vermeidung einer Verdrillung des Stents (1; 1') über seine Länge ausgebildet und angeordnet sind.

16. Katheteranordnung mit einem Katheter (7; 7''') und einem Stent nach einem der vorhergehenden Ansprüche, wobei der Katheter ein distales Ende aufweist, in dessen Bereich eine Hülleinrichtung (9; 9''') zur Aufnahme des Stents (1; 1'; 1"; 1''') in seinem ersten Zustand vorgesehen ist, sowie eine Einrichtung zum Erzeugen der Relativbewegung zwischen der Hülleinrichtung (9; 9''') und dem Stent (1; 1'; 1"; 1''') in einer ersten Richtung (5; 5'; 5"; 5''') aufweist.
